# EUROPEAN PATENT SPECIFICATION

(11) **EP 1 064 943 B1**
(45) Date of publication and mention of the grant of the patent: **09.02.2005**
(21) Application number: 99830415.8
(22) Date of filing: 30.06.1999
(51) Int. Cl.: A61K 31/405, A61K 31/22, A61K 31/205, A61P 3/06

(54) **Combination having antilipemic activity substantially free from toxic or side effects due to antilipemic drugs**
Lipidsenkende Kombination, die im wesentlichen frei ist von toxischen Wirkungen und Nebenwirkungen der Lipidsenker
Combinaison ayant une activité hypolipémiante substantiellement dénuée des effets toxiques ou effets secondaires causés par les médicaments à activité hypolipémiante

(43) Date of publication of application: 03.01.2001
(73) Proprietor: SIGMA-TAU Industrie Farmaceutiche Riunite S.p.A., 00144 Roma (IT)
(72) Inventor: Arduini, Arduino, 00135 Rome (IT); Peschechera, Alessandro, 00122 Ostia Lido (Rome) (IT); Carminati, Paolo, 20121 Milan (IT)

(56) References cited:
- EP-A- 0 383 432
- WO-A-99/01126
- DATABASE MEDLINE [Online] NLM, Bethesda, USA retrieved from DIALOG, accession no. 07446723 Database accession no. 92217262 XP002081551 & SAVICA ET AL.: "[The hypotriglyceridemic action of the combination of L-carnitine + simvastatin vs. L-carnitine and vs. simvastatin]" CLIN. TER., vol. 140, no. 1 Pt 2, January 1992 (1992-01), page 17-22
- PATENT ABSTRACTS OF JAPAN vol. 011, no. 353 (C-457), 18 November 1987 (1987-11-18) & JP 62 126126 A (SNOW BRAND MILK PROD CO LTD), 8 June 1987 (1987-06-08)
- BHUIYAN ET AL.: "The effects of 3-Hydroxy-3-Methylglutaryl-CoA reductase inhibition on tissue levels of carnitine and carnitine acyltransferase activity in the rabbit" LIPIDS, vol. 31, no. 8, 1996, pages 867-870, XP000872223

## Description

The invention described herein relates to a pharmaceutical composition comprising a statin and L-carnitine or one of its alkanoyl derivatives, useful for the prevention and treatment of statin-induced toxic or side effects.

Cardiovascular diseases related to lipid metabolism disorders are very frequent in the industrialised countries. In Italy, for instance, they account for more than 40% of the overall mortality (Capocaccia R., Farchi G., Prati S. et al.: La mortalità' in Italia nell'anno 1989. Rapporto ISTISAN 1992/22). Our knowledge of the relationships between cholesterol and coronary heart disease stems from epidemiological studies conducted in recent years. The conclusions of these studies indicate that the development of severe coronary atherosclerosis is closely related to serum cholesterol levels (McGill H.C. Jr. et al.: The International Atherosclerosis Project. Lab. Invest. 18: 463-653, 1968; Keys A.: Seven Countries: Death and Coronary Heart Disease. Harvard University Press, Cambridge, 1980).

Correction of eating habits through an appropriate diet is always the first measure to be adopted in cases of hyperlipidaemia. Good results, however, are not always achieved owing to widespread intolerance of the strict dietary regimen, to the severity of the hypercholesterolaemia or to genetic-type resistance.

In these cases, to achieve the desired results, that is to say to restore normal blood levels of triglycerides and cholesterol, it proves necessary to resort to pharmacological treatment with lipid-lowering drugs. This category includes both drugs that prevalently reduce cholesterol levels and drugs that prevalently reduce triglyceride levels.

The former group of drugs includes statins, probucol and resins, and the latter group fibrates, nicotinic acid and omega-3-series fatty acids.

The statins (simvastatin, lovastatin, pravastatin, fluvastatin and the like) are hydroxy-methylglutaryl coenzyme A (HMG-CoA) reductase inhibitors. By inhibiting this enzyme, they reduce the hepatic synthesis of cholesterol (Lancet 1994; 334: 1383-1389). To compensate for the reduction in intracellular cholesterol, the liver cell produces more receptors for lipoproteins of the LDL and VLDL series, which in this way are removed from the bloodstream.

In addition, the statins cause less absorption of cholesterol of dietary origin in the intestine and a reduced output of apoprotein B present in low-density lipoproteins (LDL).

The statins are better tolerated than the other cholesterol-lowering agents, but present certain drawbacks: the most common side effects caused by these drugs are gastrointestinal disorders, skin rashes and headache.

A number of patients have complained of sleep disorders (EJ Schaffer, N Engl J Med, 319:1222,1988; Lancet, 339:547, 29 February 1992), while significant increases in transaminase activity (GOT and GPT) and in CK compared to basal values have been observed in patients taking statins in doses of 40 mg/kg (Schweiz Med Wochenschr 1991 Jun 29; 121 (26) : 977-83).

Moreover, patients treated with simvastatin present side effects related to myopathy, rhabdomyolysis, muscle pain and increases in serum CK and LDH activity [Dedlypere J.P. & Vermeulen A. (1991) Ann. Intern. Med. 114:342; Bizzarro N. et al. (1992) Clin. Chem. 38: 1504].

EP 0383432 describes the combination of an HMG-CoA reductase inhibitor and coenzyme Q10 for the treatment of skeletal muscle myopathy caused by statins.

It has been reported that statins cause a reduction in the number of deaths due to coronary heart disease, but, on the other hand, an increase in deaths due to other events such as tumours or trauma has been noted in treated patients (Davey-Smith G., Song F., Sheldon T. A., Cholesterol lowering and mortality: the importance of considering initial level at risk. *BMJ* 1993; 306: 1367-1373; Ravnshov U.; Cholesterol lowering trials in coronary heart disease: frequency of citation and outcome. *BMJ* 1992; 305: 15-19). Young rats treated with different cholesterol-lowering agents (simvastatin, lovastatin, and pravastatin) show signs of myopathy, when high doses of simvastatin are used (Reijneveld J. C. et al., 1976 Pediatr. Res. 39: 1028-1035). Moreover, Bhuiyan et al. (Bhuiyan J. & Seccombe D.W. 1996 Lipids 31: 867-870) have demonstrated that the administration of lovastatin to rabbits causes a significant reduction in hepatic, cardiac and skeletal muscle L-carnitine.

The results of experiments in animals and in human subjects have suggested that, to reduce cholesterol levels, pharmacological treatment with statins should be used only in patients at high risk of coronary disease in the short term (JAMA, 1996; 275: 55-60).

Equally well known are the triglyceride- and cholesterol-lowering effects of a number of alkanoyl carnitines, in particular of acetyl L-carnitine. US Patent 4,268,524 describes a therapeutic method for increasing the level of high-density lipoproteins (HDL) so as to selectively reduce the LDL + VLDL:HDL ratio in the plasma of patients at risk of cardiovascular disease, in whom this ratio is abnormally high. This method includes the daily administration of 5-50 mg/kg of alkanoyl carnitine or of one of its pharmacologically acceptable salts.

The international patent application WO99/01126 filed in the name of the applicant describes the use of alkanoyl L-carnitine in combination with statins for the treatment of diseases related to lipid metabolism disorders. WO99/01126 does not describe or suggest that L-carnitine or the alkanoyl L-carnitines exert a protective action on statin-induced toxic or side effects.

It has now unexpectedly been found that the co-ordinated use - this term will be defined precisely here below - of L-carnitine or an alkanoyl L-carnitine, in which the linear or branched alkanoyl has 2-6 carbon atoms, or one of their pharmacologically acceptable salts and a statin affords a protective action against statin-induced toxic or side effects.

The well-known lack of toxic and side effects of L-carnitine and of the alkanoyl L-carnitines and the protective action exerted by these compounds on statin-induced toxic or side effects allow the statins to be used at doses higher than those usually administered (10-20 mg/day).

The co-ordinated use according to the invention is particularly useful and safe for the treatment of both hypercholesterolaemic and/or hyper-triglyceridaemic patients at high risk for cardiovascular disease in the short, medium or long term.

Thanks to the protective effect exerted by L-carnitine or by the alkanoyl L-carnitines, it has been found, in fact, that it is possible to use higher doses of statin than those normally used in human therapy, while the dose of L-carnitine or alkanoyl L-carnitines may be 100-3000 mg/day.

In the context of the invention described herein, what is meant by "co-ordinated use" of the above-mentioned compounds is, indifferently, either (i) co-administration, i.e. the substantially simultaneous administra-tion of L-carnitine or one of the aforesaid alkanoyl L-carnitines or one of their pharmacologically acceptable salts and of a statin, or (ii) the administration of a composition comprising the aforesaid active ingredients in combination and in a mixture, in addition to possible excipients. What is meant by co-administration is also a pack or manufactured article, comprising distinct administration forms of L-carnitine or one of the aforesaid alkanoyl L-carnitines, or one of their pharmacologically acceptable salts and a statin, accompanied by instructions for the co-ordinated simultaneous intake of the active ingredients according to a dosage regimen established by the primary care physician on the basis of the patient's condition.

The invention described herein relates to the use of L-carnitine or an alkanoyl L-carnitine in which the linear or branched alkanoyl has 2-6 carbon atoms, or of one of their pharmacologically acceptable salts, for the preparation of a medicinal agent useful for the treatment of statin-induced toxic or side effects.

The statin is preferably selected from the group consisting of lovastatin, simvastatin, pravastatin and fluvastatin, while the alkanoyl L-carnitine is preferably selected from the group consisting of acetyl L-carnitine, propionyl L-carnitine, butyryl L-carnitine, valeryl L-carnitine and isovaleryl L-carnitine or one of their pharmacologically acceptable salts.

More preferably, the statin is simvastatin and the alkanoyl L-carnitine is propionyl L-carnitine or one of its pharmacologically acceptable salts.

Even more preferably, the statin is simvastatin and the carnitine is L-carnitine or one of its pharmacologically acceptable salts.

What is meant by a pharmacologically acceptable salt of an alkanoyl L-carnitine is any salt of this with an acid which does not give rise to toxic or side effects. These acids are well known to pharmacologists and to experts in pharmaceutical technology.

Examples of pharmaceutically acceptable salts of alkanoyl L-carnitines, though not exclusively these, are chloride, bromide, orotate, acid aspartate, acid citrate, acid phosphate, fumarate and acid fumarate, maleate and acid maleate, mucate, acid oxalate, acid sulphate, glucose phosphate, tartrate and acid tartrate.

By favouring the use of larger doses of statins, the combination according to the invention allows better treatment of diseases related to lipid metabolism disorders, thus achieving greater therapeutic success.

The combination according to the invention also contributes to the healing and to prolonging the lives of the patients treated, amongst other things thanks to the increase in therapeutic success rates due to the possibility of maintaining the scheduled treatment protocols for longer periods, without having to discontinue the treatment owing to the toxic or side effects of the statins.

The protective action of L-carnitine or of an alkanoyl L-carnitine on the toxic or side effects of statins has been confirmed by the results of experimental studies, which are reported here below.

Though reference is made in these examples only to L-carnitine, it must, however, be understood that such protection is also afforded by the above-mentioned alkanoyl L-carnitines and by their pharmacologically acceptable salts.

### Example 1

Male Wister rats aged 23 days and weighing 45-50 g were used. The animals were housed in polycarbonate cages, 5 animals per cage, maintained at a constant temperature of 22 ± 2°C and at 55 ± 15% relative humidity, with a light-darkness cycle of 12 hours, fed on 4RF21 pellet feed (Mucedola), with tap water to drink ad *libitum.*

The control group consisted of 14 animals, whereas the groups treated with simvastatin at various doses and with simvastatin plus L-carnitine consisted of 10 animals each, according to the following experimental design:
- Control (no treatment);
- Simvastatin 70 mg/kg;
- Simvastatin 140 mg/kg;
- Simvastatin 210 mg/kg;
- Simvastatin 70 mg/kg + L-carnitine 400 mg/kg.
- Simvastatin 140 mg/kg + L-carnitine 400 mg/kg.
- Simvastatin 210 mg/kg + L-carnitine 400 mg/kg.

L-carnitine was given by oral administration via a gastric tube, twice daily (2 x 200 mg/kg) suspended in 0.5% carboxymethylcellulose (CMC) to the groups treated with statin, or in water when administered alone.

Simvastatin was administered orally suspended in 0.5% carboxy-methylcellulose (CMC) (10 mL/kg).

The duration of the treatment was 9 days.

24 hours after the last treatment, the animals were anaesthetised and blood samples were taken from the sublingual vein.

The blood was centrifuged at 400 rpm for 30 min and the serum thus obtained was used to evaluate plasma levels of CK, GOT, GPT and cholesterol.

The CK, GOT, GPT and cholesterol analyses were carried out using a Cobas Mira S (Roche) automatic analyser and Roche diagnostic kits.

Since the plasma enzyme activity showed a highly skewed distribution, it was decided to analyse the data using the non-parametric Mann-Whitney U test; the test data are shown as median values together with the associated ranges.

The results obtained are reported in Table 1.

**Table 1**

| | | GOT | GPT | CK | Cholesterol |
|---|---|---|---|---|---|
| | | U/L | U/L | U/L | mg/dl |
| Control | Median | 140.73 | 45.94 | 1056.12 | 73.18 |
| | Range | 96.4-196.4 | 40.3-57.4 | 477.7-1616.4 | 60.3-80.7 |
| Simvastatin 70 mg/kg | Median | 125.68 | 50.65 | 971.51 | 71.35 |
| | Range | 98.8-237.1 | 41.1-73.6 | 396.8-1754.2 | 44.6-87.1 |
| Simvastatin 140 mg/kg | Median | 189.97*** | 69.18*** | 1308.92 | 56.72 |
| | Range | 139.0-293.8 | 40.7-98.7 | 417.8-1836.1 | 44.2-81.3 |
| Simvastatin 210 mg/kg | Mediana | 686.69*** | 93.71** | 1784.32* | 46.54*** |
| | Range | 172.3-5288.8 | 35.5-494.9 | 8808-4887.0 | 35.9-66.1 |
| Simvastatin 70 mg/kg + | Mediana | 123.14 | 50.42 | 709.29 | 74.95 |
| L-carnitine 200 mg/kg | Range | 100.4-204.4 | 37.8-76.6 | 442.0-1840.1 | 56.7-119.4 |
| Simvastatin 140 mg/kg + | Median | 209.45 | 57.49 | 762.53* | 57.21 |
| L-carnitine 200 mg/kg | Range | 112.3-373.6 | 42.3-138.0 | 350.2-1455.7 | 33.2-75.3 |
| Simvastatin 210 mg/kg + | Median | 435.11 | 77.80 | 741.65* | 40.06 |
| L-carnitine 200 mg/kg | Range | 134.0-2422.7 | 51.8-494.9 | 535.2-2425.0 | 31.3-66.5 |

Mann-Whitney U test:
- significance: *** = p<0.002; ** = p<0.02; * = p<0.05;
- the significance of the groups treated with statin (alone) was calculated versus the control group;
- the significance of the groups treated with statin in combination with

L-carnitine was calculated versus the group treated with statin alone.

The results presented in Table 1 indicate that the administration of simvastatin at the highest dose (210 mg/kg) caused a substantial and significant increase in plasma GOT (p<0.002), GPT (p<0.002) and CK (p<0.05) compared to controls. At a lower dose (140 mg/kg), simvastatin treatment caused an increase in all the enzyme activities tested, with GOT (p<0.002) and GPT (p<0.02).

Simvastatin treatment at the lowest dose (70 mg/kg) did not significantly increase the enzyme activity tested.

The cholesterol level was significantly lowered only at the highest simvastatin dose used.

The administration of L-carnitine to the groups treated with simvastatin showed lower plasma CK activity compared to the group treated with simvastatin alone. Statistically, L-carnitine administration was significantly effective in counterbalancing the plasma CK elevation at the simvastatin doses of 140 and 210 mg/kg (Table 1).

Simvastatin treatment at the lowest dose (70 mg/kg) in combination with L-carnitine reduced plasma CK activity as compared to simvastatin alone at the same dose, though not to a statistically significant extent.

The results of these studies furnish substantial evidence of the protective action of L-carnitine and of the alkanoyl L-carnitines on the toxic and side effects of statins which constitutes the basis for the invention described herein.

In a second experiment conducted in the same way as the first, the only difference being that L-carnitine was administered in the animals' drinking water, comparable results were obtained.

Therefore, a further realisation of the invention described herein comprises the co-ordinated use of L-carnitine or one of its alkanoyl derivatives or one of their pharmacologically acceptable salts and of a statin according to the above definitions, in the treatment of animals, such as, for example, livestock and, particularly, pets. In this particular realisation, L-carnitine, or one of its derivatives, may be in solid form, such as, for example, fumarate, tartrate or mucate, to be dissolved in drinking water, or in metered-dose liquid form, to be diluted.

## Claims

1. Use of L-carnitine or an alkanoyl L-carnitine, in which the linear or branched alkanoyl has 2-6 carbon atoms, or of one of their pharmacologically acceptable salts, for the preparation of a medicinal agent useful for the treatment of HMG-COA reductase inhibitor-induced toxic or side effects.

2. Use according to Claim 1, in which the amount of the HUG-COMA reductase inhibitor is higher than 20 mg/day.

3. Use according to Claim 1, in which the HMG-COA reductase inhibitor is selected from the group consisting of lovastatin, simvastatin, pravastatin and fluvastatin.

4. Use according to Claim 3, in which the HMG-COA reductase inhibitor is simvastatin.

5. Use according to Claim 1, in which the alkanoyl L-carnitine is selected from the group consisting of acetyl L-carnitine, propionyl L-carnitine, butyryl L-carnitine, valeryl L-carnitine, and isovaleryl L-carnitine.

6. Use according to Claim 5, in which the alkanoyl L-carnitine is propionyl L-carnitine.

7. Use according to Claim 1, in which L-carnitine is present.

8. Use according to Claim 1, in which the pharmacologically acceptable salt of L-carnitine or of the alkanoyl L-carnitines is selected from the group consisting of chloride, bromide, orotate, acid aspartate, acid citrate, acid phosphate, fumarate and acid fumarate, maleate and acid maleate, mucate, acid oxalate, acid sulphate, glucose phosphate, tartrate and acid tartrate.

9. Use according to Claim 1, in which the administration is sequential.

10. Use according to Claim 1, in which the administration is substantially simultaneous.

## Patentansprüche

1. Verwendung von L-Carnitin oder einem Alkanoyl-L-carnitin, worin das lineare oder verzweigte Alkanoyl 2 bis 6 Kohlenstoffatome hat, oder eines der pharmakologisch akzeptablen Salze davon zur Erzeugung eines medizinischen Mittels zur Behandlung von HMG-CoA-Reduktaseinhibitor-induzierten toxischen oder Nebenwirkungen.

2. Verwendung nach Anspruch 1, worin die Menge des HMG-CoA-Reduktaseinhibitors höher als 20 mg/Tag ist.

3. Verwendung nach Anspruch 1, worin der HMG-CoA-Reduktaseinhibitor ausgewählt ist aus der Gruppe bestehend aus Lovastatin, Simvastatin, Pravastatin und Fluvastatin.

4. Verwendung nach Anspruch 3, worin der HMG-CoA-Reduktaseinhibitor Simvastatin ist.

5. Verwendung nach Anspruch 1, worin das Alkanoyl-L-carnitin ausgewählt ist aus der Gruppe bestehend aus Acetyl-L-carnitin, Propionyl-L-carnitin, Butyryl-L-carnitin, Valeryl-L-carnitin und Isovaleryl-L-carnitin.

6. Verwendung nach Anspruch 5, worin das Alkanoyl-L-carnitin Propionyl-L-carnitin ist.

7. Verwendung nach Anspruch 1, worin L-Carnitin vorhanden ist.

8. Verwendung nach Anspruch 1, worin das pharmakologisch akzeptable Salz von L-Carnitin oder den Alkanoyl-L-carnitinen ausgewählt ist aus der Gruppe bestehend aus Chlorid, Bromid, Orotat, saurem Aspartat, saurem Citrat, saurem Phosphat, Fumarat und saurem Fumarat, Maleat und saurem Maleat, Mucat, saurem Oxalat, saurem Sulfat, Glucosephosphat, Tartrat und saurem Tartrat.

9. Verwendung nach Anspruch 1, worin die Verabreichung aufeinanderfolgend ist.

10. Verwendung nach Anspruch 1, worin die Verabreichung im wesentlichen gleichzeitig ist.

## Revendications

1. Utilisation de la L-carnitine ou d'une L-carnitine d'alcanoyle dans laquelle l'alcanoyle linéaire ou ramifié présente 2 à 6 atomes de carbone, ou d'un de leurs sels pharmacologiquement acceptables, pour la préparation d'un agent médicinal utilisable dans le traitement des effets toxiques ou secondaires induits par un inhibiteur de la HMG-COA réductase.

2. Utilisation selon la revendication 1, dans laquelle la quantité de l'inhibiteur de la HMG-COA réductase est supérieure à 20 mg/jour.

3. Utilisation selon la revendication 1, dans laquelle l'inhibiteur de la HMG-COA réductase est choisi dans le groupe consistant en la lovastatine, la simvastatine, la pravastatine et la fluvastatine.

4. Utilisation selon la revendication 3, dans laquelle l'inhibiteur de la HMG-COA réductase est la simvastatine.

5. Utilisation selon la revendication 1, dans laquelle la L-carnitine d'alcanoyle est choisie dans le groupe consistant en la L-carnitine d'acétyle, la L-carnitine de propionyle, la L-carnitine de butyryle, la L-carnitine de valéryle, et la L-carnitine d'isovaléryle.

6. Utilisation selon la revendication 5, dans laquelle la L-carnitine d'alcanoyle est la L-carnitine de propionyle.

7. Utilisation selon la revendication 1, dans laquelle la L-carnitine est présente.

8. Utilisation selon la revendication 1, dans laquelle le sel pharmacologiquement acceptable de la L-carnitine ou des L-carnitines d'alcanoyle est choisi dans le groupe consistant en le chlorure, le bromure, l'orotate, l'aspartate acide, le citrate acide, le phosphate acide, le fumarate et le fumarate acide, le maléate et le maléate acide, le mucate, l'oxalate acide, le sulfate acide, le phosphate de glucose, le tartrate et le tartrate acide.

9. Utilisation selon la revendication 1, dans laquelle l'administration est séquentielle.

10. Utilisation selon la revendication 1, dans laquelle l'administration est essentiellement simultanée.
